# EUROPÄISCHE PATENTSCHRIFT

(11) **EP 1 121 925 B1**
(45) Veröffentlichungstag und Bekanntmachung des Hinweises auf die Patenterteilung: **17.11.2004**
(21) Anmeldenummer: 01101790.2
(22) Anmeldetag: 26.01.2001
(51) Int. Cl.: A61K 7/06, A61K 7/48

(54) **Deodorierende Haarpflegemittel**
Deodorizing hair care composition
Composition désodorisant pour le soin des cheveux

(30) Priorität: 04.02.2000 DE 10005018
(43) Veröffentlichungstag der Anmeldung: 08.08.2001
(73) Patentinhaber: Henkel Kommanditgesellschaft auf Aktien, 40589 Düsseldorf (DE)
(72) Erfinder: Bernecker, Ullrich, Dr., 52393 Hürtgenwald (DE); Hollenberg, Detlef, Dr., 40699 Erkrath (DE); Bossmann, Britta, 40699 Erkrath (DE)

(56) Entgegenhaltungen:
- WO-A-96/36312
- FR-A- 2 752 730
- GB-A- 2 140 452

## Beschreibung

Die Erfindung betrifft ein Verfahren zur Pflege des Haars und zur Verringerung der dem Haar anhaftenden Körper- und Umweltgerüche durch Behandlung mit einem Pflegemittel, das einen wäßrigen oder wäßrig-alkoholischen Extrakt von Blättern, Rinde oder Wurzeln der Birkenpflanzen enthält.

Es sind zahlreiche kosmetische Produkte zur Verringerung oder Hemmung von Körpergeruch bekannt. Diese beruhen entweder auf Duftstoffen zur Geruchsüberdeckung, auf Adsorbentien zur Geruchsbindung, auf antimikrobiellen Stoffen zur Hemmung der schweißzersetzenden Hautkeime, auf Enzyminhibitoren zur Hemmung der schweißspaltenden Esterasen, oder auf Stoffen, die der Schweißbildung entgegenwirken, den sogenannten Antitranspirantlen.

Unangenehmer Körpergeruch wird aber nicht nur durch die Schweißabsonderung oder Schweißzersetzung erzeugt, sondern kann auch durch das Anhaften von Gerüchen aus der Umwelt an der Haut und insbesondere am Haar bedingt sein, das aufgrund seiner großen Oberfläche anfällig für die Aufnahme und Fixierung unangenehmer Umweltgerüche ist. So nimmt das Haar durch bloßen Aufenthalt in Räumen, in denen stark geraucht wird, einen penetranten "Kneipengeruch" an. Auch der Aufenthalt in Küchen, in denen stark riechende Speisen zubereitet werden, in Werkstätten oder Garagen, in denen die Luft mit Mineralöl und Dieseltreibstoff verunreinigt ist, oder in Viehställen führt zu einer Aufnahme typischer Gerüche, die besonders hartnäckig am Haar anhaften. Auch durch Dauerwellbehandlung kann das Haar einen unangenehmen Geruch annehmen.

Zwar kann man durch Waschen der Haare mit den üblichen Shampoos diese Gerüche weitgehend aus dem Haar entfernen, es besteht jedoch ein Bedürfnis, den unangenehmen Geruch der Haare durch eine weniger aufwendige Pflegebehandlung wirksam zu bekampfen.

So sind z. B. bisher lediglich Parfümsprays zur Überdeckung von Gerüchen bekannt. Wirksame "leave-on"-Präparate, die z. B. als Spray oder Pflegeschaum oder als Haarwasser auf dem Haar verteilt werden können und dabei anhaftende Gerüche wirksam neutralisieren oder inhibieren, sind bisher nicht bekannt geworden.

Das aus Oberflächenverletzungen der Birke austretende Sekret (Birkenwasser) und auch Extrakte aus Pflanzenteilen der Birke sind als Komponenten in Haar- und Kopfhautpflegemitteln seit langem bekannt. Aus GB 2140 452 war die Verwendung von Birkenextrakten auch in Haarwaschmitteln bekannt. In JP 93/066144 B werden Extrakte der Weißbirke und anderer Pflanzen auch als Deodorantien für die Mundhöhle empfohlen.

Aus WO96/3631 A1 ist die Kombination eines Birkenextrakts und eines polymeren Konditioniermittels, das die Monomereinheiten Acrylsäure, Acrylamid, Dimethyldiallylammoniumchlorid oder Mischungen davon umfasst, in Haarpflegemitteln bekannt. Durch diese Kombination wird die Bildung freier Radikale bei längerer Lagerung des Haarpflegemittels vermindert.

Es wurde nun festgestellt, daß wäßrige oder wäßrig-alkoholische Extrakte aus Pflanzenteilen der Birke sich zur Deodorierung der Haut und insbesondere der Haare eignen.

Gegenstand der Erfindung ist also ein Verfahren zur Pflege der Haut oder der Haare und zur Verringerung von Körper- und Umweltgerüchen der Haare durch Behandlung mit einem Pflegemittel, das nicht unmittelbar nach der Anwendung mit Wasser abgespült wird und das einen wäßrigen oder wäßrig-alkoholischen Extrakt aus Pflanzenteilen der Birke in einer Menge von wenigstens 0,01 Gew.-% wasserfreie Trockenmasse enthält.

Als Pflanzenteile, die für die Erfindung brauchbare Extrakte liefern, sind Blätter, Rinde, Xylem und Wurzeln zu nennen. Bevorzugt ist der Extrakt aus Blättern und Rinde. Die Extrakte zeichnen sich durch einen hohen Gehalt an Flavonoiden, z. B. von Hyperosid aus, die eine Wirksamkeit gegen freie Radikale und eine antioxidative und schützende Wirkung auf die Zellmembran aufweisen. Obwohl die Extrakte aus Blättern der Spezies Betula alba (L.) bevorzugt sind, eignen sich auch Extrakte aus Blättern oder Rinde anderer Spezies der Pflanzenfamilie der Betulaceae. Solche Extrakte sind im Handel erhältlich, z. B. unter dem Warenzeichen Herbasol® -Extrakt Birke (Cosmetochem) mit ca. 3,5 bis 5,5 Gew.-% Trockenmasse in einem Lösungsmittelgemisch aus Wasser und Propylenglycol (58:42). Als wäßrig-alkoholische Extrakte sind solche gemeint, die als Extraktionsmittel und flüssige Trägerkomponente Wasser und einoder mehrwertige Alkohole mit 2-6 C-Atomen enthalten. Das Mengenverhältnis von Wasser und Alkoholen kann dabei von 1:0,1 bis 1:10 liegen. Geeignete Alkohole sind z. B. Ethanol, Isopropanol, Ethylenglycol, 1,2-Propylenglycol, Glycerin, Diethylglycol, Butylenglycol, Ethyldiglycol und Gemische solcher Alkohole.

Das Pflegemittel kann z. B. eine wäßrige oder wäßrig-alkoholische Lotion, eine Emulsion, ein Gel, ein Stiftpräparat, ein Spray oder ein Schaumkonzentrat sein. In einer bevorzugten Ausführungsform wird für das erfindungsgemäße Verfahren als Pflegemittel ein wäßriger Spray oder ein wäßriger Pflegeschaum verwendet.

Als Spray eignen sich dabei wäßrige oder wäßrig-alkoholische Zubereitungen niedriger Viskosität, die sich entweder unter Verwendung geeigneter Aerosol-Druckgase aus Aerosolflaschen versprühen oder vernebeln lassen oder die aus Behältem mit einem Pumpmechanismus in Form feiner Tröpfchen auf die Haut oder auf das Haar aufgebraucht werden können.

Als wäßrige Pflegeschäume werden Zubereitungen verstanden, die schaumbildende Komponenten enthalten und entweder unter Verwendung geeigneter Aerosol-Druckgase in Druckgasverpackungen mit Schaumventil verpackt werden und aus diesen als feinblasiger Schaum austreten, oder die aus nicht―Aerosol-Abgabebehältem mit Reservoir, Pumpmechanismus und Abgabekopf mit Luftbeimischkammer als Schaum ausgebracht werden.

Ein bevorzugter Gegenstand der vorliegenden Erfindung sind Mittel zur Pflege des Haars in Form von wäßrigen oder wäßrig-alkoholischen Zubereitungen, die ein filmbildendes Polymerisat enthalten und als deodorierende Komponente einen wäßrigen oder wäßrig-alkoholischen Extrakt von Pflanzenteilen der Birke in einer Menge von wenigstens 0,01 Gew.-% Trockenmasse enthalten.

Als wäßrig-alkoholische Zubereitungen werden dabei solche verstanden, die als flüssige Trägerkomponente Wasser und ein- oder mehrwertige Alkohole mit 2-6 C-Atomen enthalten. Das Mengenverhältnis von Wasser und Alkohol kann dabei von 1:0,1 bis 1:10 liegen. Geeignete Alkohole sind z. B. Ethanol, Isopropanol, Ethylenglycol, 1,2-Propylenglycol, Glycerin, Diethylenglycol, Butylenglycol, Butylglycol, Ethyldiglycol und Gemische solcher Alkohole.

Durch den Gehalt an filmbildenden Polymerisaten wird das Haar leicht gefestigt und der Halt der Frisur erhöht. Auch wird der deodorierende Wirkstoff fester an die Haaroberfläche gebunden und die Wirkung verlängert.

Als filmbildende Polymere eignen sich alle in dem Trägermedium löslichen Polymerisate mit filmbildenden Eigenschaften, d.h. mit mittleren Molekulargewichten von wenigstens 10 000 D. Beispiele solcher filmbildenden Polymere sind z. B. Polyvinylpyrrolidone und Mischpolymerisate aus Vinylpyrrolidon und Vinylacetat, Mischpolymerisate aus Vinylacetat und Crotonsäure, Copolymere aus Methyl-Vinylether und Maleinsäureanhydrid, quaternierte Copolymere aus Vinylpyrrolidon und Dimethylaminoethylmethacrylat, Copolymere aus Vinylpyrrolidon und Vinylimidazoliniummethochlorid, Copolymere aus Methylmethacrylat, tert-Butylaminoethylmethacrylat, 2-Hydroxypropylmethacrylat und Isooctylacrylamid und andere. Solche filmbildenden Polymerisate sind in den erfindungsgemäßen Haarpflegemitteln bevorzugt in Mengen von wenigstens 0,1 Gew.-%, insbesondere von 0,2-2 Gew.-% enthalten.

Zur Verbesserung der Filmeigenschaften können die erfindungsgemäßen Zubereitungen außerdem kationische Tenside, kationische oder zwitterionische Polymere, Proteinhydrolysate und/oder weichmachende Komponenten, z. B. Polyethylenglycole, oder mikroemulgierte Ölkomponenten enthalten.

Als kationische Tenside sind bevorzugt quartäre Ammoniumverbindungen mit einer oder zwei langkettigen Alkyl-, Acyloxyalkyl oder Acylamidoalkylgruppen geeignet. Beispiele solcher quartärer Ammoniumverbindungen sind z. B. Cetyltrimethylammoniumchlorid, Lauryldimethylbenzylammoniumchlorid, Cetylpyridiniumchlorid, Kokosacylaminopropyl-trimethylammoniumchlorid, Distearyloxyethyl-dimethylammonium-methoxysulfat, Distearyldimethylammoniumchlorid, Distearyloxyethyl-hydroxyethylmethylammoniumchlorid, kationische Zuckerderivate wie z. B. das Lauryl-methylgluceth-10-hydroxypropyl-dimoniumchlorid (Glucquat®100) oder die quaternierten Proteinhydrolysate wie z. B. das Handelsprodukt Lamequat®L. Geeignete kationische Tenside sind auch kationische Silicone wie z. B. die Handelsprodukte DOW CORNING Q2-7224 (stabilisiertes Trimethylsilylamodimethicon) DOW CORNING 929 Emulsion (hydroxylamino-modifiziertes Silikon, auch als Amodimethicon bezeichnet), SM-2059 (General Electric), SLM-55067 (Wacker Chemie) oder Abil®-Quat 3270 und 3272 (Th. Goldschmidt).

Auch nichtquaternierte, tertiäre Aminoverbindungen eignen sich als kationische Tenside, da sie mit Säuren kationische Ammoniumgruppen ausbilden. Als geeignetes Beispiel sei Stearylamidopropyldimethylamin (Tego Amid®S 18) genannt. Als kationische Polymere eigenen sich z. B. quaternierte Celluloseether, Polysiloxane mit quartären Ammoniumgruppen, Polydimethyldiallylammoniumchlorid, Copolymere aus Acrylamid und Dimethyldiallylammoniumchlorid, Copolymere aus Vinylpyrrolidon und Dimethylaminoethylmethacrylat, quaterniert mit Diethylsulfat und andere. Zwitterionische Polymere sind z. B. Copolymere aus (Meth)acrylamidopropyl-trimethylammoniumchlorid und (Meth)acryl- oder Crotonsäure.

Zur Formulierung wäßriger Pflegeschäume ist darüberhinaus ein Gehalt an schaumbildenden Tensiden, bevorzugt amphoteren, zwitterionischen oder nichtionischen Tensiden oder Gemischen davon, zu empfehlen.

Geeignete nichtionische Tenside sind z. B. die Alkyl-(oligo)glucoside, die durch Umsetzung von Alkoholen mit 8-16 C-Atomen mit z. B. Butylglucosid durch Transacetalisierung oder durch direkte Acetalisierung aus Glucose und Fettalkohol zugänglich sind und der Formel RO-(Z)ₓ entsprechen, in der R eine C₈-C₁₆-Alkylgruppe und Z ein Monosaccharidrest, insbesondere ein Glucoserest, und x dessen mittlerer Oligomerisationsgrad, eine Zahl von 1-5, bevorzugt von 1-2, darstellt. Weitere geeignete nichtionogene Tenside sind z. B.
- Anlagerungsprodukte von 2-30 Mol Ethylenoxid und/oder von 1-5 Mol Propylenoxid an lineare Fettalkohole mit 8-18 C-Atomen, an Fettsäuren mit 12-18 C-Atomen, an Fettsäuremonoglyceride von C₁₂-C₁₈-Fettsäuren, an Sorbitanmonofettsäureester von Fettsäuren mit 12-18- C-Atomen, an Fettsäurealkanolamide, an Methylglucosid-Fettsäureester, an gehärtetes Rizinusöl und an andere Lipide mit beweglichen Wasserstoffatomen.
- Aminoxid-Tenside, z. B. Alkylaminoxide mit 12-18- C-Atomen und Acylamidopropyl-dimethylaminoxid mit 12-18- C-Atomen in der Acylgruppe.

Auch selbst nicht wasserlösliche Tenside, die aber in Gegenwart wasserlöslicher Tenside solubilisiert werden und dann sowohl zur Viskosität und zur Verdickbarkeit durch Elektrolyte als auch zur Feinblasigkeit und Cremigkeit des Schaumes beitragen, könne in Mengen bis zu 5 Gew.-% in den erfindungsgemäßen Haarreinigungsmitteln enthalten sein. Solche Produkte sind z. B.
- Fettsäuremonoethanolamide, Fettsäurediethanolamide und Fettsäuremonöisopropanolamide von C₁₂-C₁₈ Fettsäuren.
- Fettsäurepartialglyceride (Monoglyceride und Mono-/Diglyceridgemische) und
- Sorbitanmono- und -difettsäureester

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, H-Hydroxyethyl-N-alkylamidopropylglycine, N-Alkyltaurine, N-Alkylsarcosine und Alkylaminoessigsäuren mit jeweils etwa 8-18 C-Atomen in der Alkylgruppe. Besonders bevorzugte ampholytische Tenside sind das N-Kokosalkylaminopropionat und das Kokosacylaminoethylaminopropionat.

Als zwitterionische Tenside werden solche oberflächenaktiven Verbindungen bezeichnet, die im Molekül mindestenes eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Darüber hinaus können die erfindungsgemäßen Pflegemittel Öl- und Fettkomponenten in emulgierter Form enthalten. Geeignete Ölkomponenten sind z. B. pflanzliche und tierische Triglyceridöle wie z. B. Olivenöl, Sojaöl, Sonnenblumenöl, Rapsöl, Kokosöl, Palmöl, Sesamöl, Mandelöl, Nachtkerzenöl, Sheabutter, oder Fettsäure-Fettalkoholester oder Wachsester, wie sie im Jojobaöl oder im Spermöl vorliegen. Auch synthetische Ölkomponenten, insbesondere die Ester von Fettsäuren oder Fettalkoholen wie z. B. Butylstearat, Isopropylmyristat, Isopropylpalmitat, Decyloleat, Hexyllaurat, Oleyloleat, Cetylpalmitat, Oleylerucat, Fettsäuremono- diglyceride sowie lineare und verzweigte Fettalkohole mit 12-22 C-Atomen wie z. B. Oleylalkohol, Cetyl- und Stearylalkohol, 2-Hexyldecanol, 2-Octyldodecanol, Isotridecanol, Wachse (z. B. Bienenwachs, Sonnenblumenwachs) eignen sich als Öl und Fettkomponenten.

Auch Kohlenwasserstoffe wie z. B. Paraffinöle, Vaseline, Squalan und synthetische Kohlenwasserstoffe wie z. B. flüssige Polyolefine oder 1,3-Di-(2-ethylhexyl)-cyclohexan, synthetische Di-n-alkylether oder Silikonöle wie z. B. die linearen Polydimethylsiloxane und die niedrigsiedenden cyclischen Silicone wie z. B. Decamethylcyclopentasiloxan oder Octamethylcylotetrasiloxan sind geeignete Ölkomponenten. Diese Öl- oder Fettkomponenten sind in emulgierter Form, also als Öl-in-Wasser Emulsion oder Mikroemulsion, in den erfindungsgemäßen Pflegemitteln enthalten.

Die zur Emulgierung eingesetzten Emulgatoren entsprechen den weiter oben genannten nichtionogenen, zwitterionischen oder kationischen Tensiden. Darüberhinuas können die erfindungsgemäßen Mittel Wirkstoffe zur Pflege der Kopfhaut und zum Schutz der Haare enthalten. Solche Stoffe sind z. B.
- Strukturanten wie z. B. Glukose oder Maleinsäure
- Hydroxycarbonsäuren (z. B. Milchsäure, Zitronensäure) oder deren Salze,
- Vitamine wie z. B. Tocopherole, Retinol oder Retinoate, Ascorbinsäure oder Ascorbate,
- Provitamine wie z. B. D-Panthenol oder Pantothenate, Biotin
- Feuchthaltemittel wie z. B. Harnstoff, Pyrrolidoncarbonsäure, Allantoin
- Lichtschutzmittel, z. B. UV-Filtersubstanzen, Titandioxid

Weitere Formulierungshilfsmittel, die zur Stabilität der Zusammensetzung oder zur Konfektionionierung beitragen, sind z. B.
- Antioxidantien
- Konservierungsmittel, z. B. p-Hydroxybenzoesäureester
- Puffersubstanzen
- Farbstoffe, Pigmente, Trübungs- oder Perlglanzmittel
- Duftstoffe

Zur Formulierung als Aerosol können Druckgase, bevorzugt verflüssigte Gase wie z. B. Propan, Butan, Dimethylether oder 1,1,1,2-Tetrafluorethan oder auch Kohlendioxid, Stickoxidul (N₂O) oder Gemische dieser Gase verwendet werden. Das Aerosol kann als Schaum oder als Sprayformulierung aufgetragen werden. Die Zusammensetzungen können auch als treibgasfreie Pflegesprays entweder mit Hilfe eines Pumpzerstäubers oder einer sogen. "squeeze bottle" angewendet werden. Zur Verwendung als treibgasfreier Pflegeschaum wird die erfindungsgemäße Zusammensetzung in einen Schaumspender mit manuell zu betätigender Pumpe abgefüllt. Solche Schaumspender sind z. B. aus US 4,957,218, US 5,339,988, WO 93/00089 und anderen Druckschriften bekannt. Die folgenden Beispiele sollen den Gegenstand der Erfindung näher erläutern.

### Beispiele

### 1. Haarpflegesprays

Es wurden folgende Konzentrate hergestellt:

| | **1** | **2** | **3** | **1V** | **2V** |
|---|---|---|---|---|---|
| Ethanol | 85 | 85 | 85 | 85 | 85 |
| Amphomer® | 0,35 | 0,35 | 0,35 | 0,35 | 0,35 |
| Luviset® CA66 | 0,50 | 0,50 | 0,50 | 0,50 | 0,50 |
| Herbasol® Extrakt Birke | 4,0 | 2,0 | 2,0 | ---- | ---- |
| Dow Corning 345 Fluid | ---- | ---- | 1,0 | ---- | 1,0 |
| Wasser | ad 100 | ad 100 | ad 100 | ad 100 | ad 100 |
| Abfüllung: 48,0 g der Rezepturen wurden in je eine 150 ml PE-Pumpensprayflasche mit M150DAV-Düse abgefüllt. | | | | | |

### 2. Haarpflegemilch

| | 1 | 2 | 3 | 4 |
|---|---|---|---|---|
| Luviskol® VA64 | 1,25 | 0,8 | 0,8 | 0,8 |
| Gafquat® 755 N | 0,60 | 1,0 | 1,0 | 1,0 |
| Dehyquart® A | 0,50 | ---- | ---- | ---- |
| Tego Amid® S18 | 0,60 | ---- | ---- | ---- |
| Armocare® VGH-70 | 0,45 | ---- | ---- | ---- |
| Sepigel® 305 | ---- | 1,3 | 1,3 | 1,3 |
| Dow Corning 949-Emulsion | ---- | 1,0 | 1,0 | 1,0 |
| Dow Corning 1401 Fluid | ---- | 2,0 | 2,0 | 2,0 |
| Ambroxan® | ---- | ---- | ---- | 0,10 |
| Dioctylcarbonat | ---- | ---- | 0,75 | ---- |
| Cetiol® OE | ---- | ---- | 0,75 | ---- |
| Ethanol | 30 | 15 | 15 | 15 |
| Herbasol® Extrakt Birke | 2,0 | 2,0 | 2,0 | 2,0 |
| Milchsäure 80 % | 0,60 | 0,60 | 0,60 | 0,60 |
| Wasser | ad 100 | ad 100 | ad 100 | ad 100 |

Es wurden folgende Handelsprodukte verwendet:
- Amphomer®(National Starch):: Copolymer aus Methylmethacrylat, tert. Butylaminoethylmethacrylat, 2-Hydroxypropylmethacrylat und N-(1,1,3,3-Tetramethylbutyl)-acrylamid
- Luviset®-CA66 (BASF):: Copolymer aus Vinylacetat und Crotonsäure
- Gafquat® 755 N (GAF):: Copolymer aus Vinylpyrrolidon und Dimethylaminoethylmethacrylat, quaterniert mit Diethylsulfat. (20%-ig in Wasser)
- Dehyquart®A (Cognis Deutschland):: Cetyl-trimethylammonium-chlorid (25%-ig in Wasser)
- Tego Amid® S18 (Tego):: Stearamidopropyl-dimethylamin
- Armocare®-VGH-70 (Akzo-Nobel):: N,N-Bis-(2-Palmitoyloxyethyl)-dimethylammoniumchlorid (70 %, Propylenglycol 22 %)
- Sepigel®305: (Seppic): Polyacrylamid, Laureth-7, C₁₃₋₁₄-Isoparaffin
- Dow-Corning 949-Emulsion:: Aminodimethicone, Cetriumonium Chloride, Trideceth-12
- Dow-Corning 1401 Fluid:: Dimethylcyclosiloxan, Dimethylpolysiloxanol
- Ambroxan® (Henkel):: 8α,12-Epoxy-13, 14, 15, 16-tetranorlabdan
- Cetiol® OE: (Cognis Deutschland): Di-n-octylether
- Herbasol® Extrakt Birke: (Cosmetochem): Birkenblätter-Extrakt, 4,5 % Trockensubstanz in Wasser/ Propylenglycol (58/42)
- Luviset®-VA64 (BASF):: Vinylacetat-Vinylpyrrolidon-Copolymer (40:60)
- Dow-Corning® 345 Fluid:: Cyclomethicone

### Haar-Deodorierungstest

### 1. Vorbehandlung des Haars

6 Strähnen von je ca. 2 g Gewicht (naturblond, Fa. Kerling) wurden für jede Versuchsreihe mit Shampoo (Polykur Aloe & Mango) und anschließend mit reiner Alkylethersulfat-Lösung (Texapon N, 12% AS) gewaschen, getrocknet und auf Geruchsneutralität geprüft.

Die gereinigten Strähnen wurden dann an einem modifizierten Dreifuß hängend befestigt und in einen Exsikkator gestellt. Der verschlossene Exsikkator wurde dann auf ein Vakuum von ca. 900 mbar evakuiert. Bei diesem Druck wurde durch einen zweiten Schlauch Luft angesaugt, durch gleichzeitige Evakuierung wurden bei konstanten Druck von 300 mbar ca. 200 l/h Luft durchgeleitet.

Nun wurde am Ende des Ansaugrohrs eine Zigarette befestigt und an einem Stativ arretiert, so daß die Ausgangsluft durch die Zigarette strömte. Schließlich wurde die Zigarette entzündet und die Pumpe abgestellt, so daß der Unterdruck des Exsikkators den Rauch bis zum Druckausgleich ansaugte.

Nach 5 Minuten wurde der Exsikkator geöffnet, die Haarsträhnen entnommen und ca. 5 Minuten bei 20°C in einem gut durchgelüfteten, rauchfreien Raum konditioniert.

### 2. Versuchsdurchführung:

Die Haarsträhnen wurden von beiden Seiten mit je 3 Pumphüben besprüht. Dies entspricht einer Menge von ca.0,8 g des Pflegemittels. Nach der Behandlung wurden die Strähnen noch 10 Minuten bei 20°C in einem rauchfreien, gut belüfteten Raum konditioniert.

### 3. Bewertung:

Ein Prüfpanel aus drei Personen beurteilte die Rauchintensität des Geruchs nach folgendem Bewertungsschema:
Kein Rauch wahrnehmbar = 0
Schwacher Rauchgeruch = 1
Deutlicher Rauchgeruch = 2
Starker Rauchgeruch = 3

Aus den Bewertungen der Prüfer wurden für jede Versuchsreihe Mittelwerte gebildet:

| | | | | | |
|---|---|---|---|---|---|
| Rezeptur | 1 | 2 | 3 | 1V | 2V |
| Rauchintensität | 0,7 | 1,5 | 1,8 | 3 | 2,3 |

## Patentansprüche

1. Verfahren zur Verringerung von Körper- und Umweltgerüchen der Haut oder der Haare durch Behandlung mit einem Pflegemittel, das nicht unmittelbar nach der Anwendung mit Wasser abgespült wird, **dadurch gekennzeichnet, daß** das Pflegemittel einen wäßrigen oder wäßrig-alkoholischen Extrakt von Pflanzenteilen der Birke in einer Menge von wenigstens 0,01 Gew.-% Trockenmasse enthält.

2. Verfahren nach Anspruch 1, **dadurch gekennzeichnet, daß** man ein Pflegemittel in Form eines wäßrigen oder wäßrig-alkoholischen Sprays oder eines wäßrigen Pflegeschaums verwendet.

3. Mittel zur Pflege des Haares und zur Verringerung von Körper- und Umweltgerüchen der Haare in Form einer wässrigen oder wässrig-alkoholischen Zubereitung, die ein filmbildendes Polymer, ausgewählt aus Polyvinylpyrrolidonen und Mischpolymerisaten aus Vinylpyrrolidon und Vinylacetat, Mischpolymerisaten aus Vinylacetat und Crotonsäure, Copolymeren aus Methyl-Vinylether und Maleinsäureanhydrid, quatemierten Copolymeren aus Vinylpyrrolidon und Dimethylaminoethylmethacrylat, Copolymeren aus Vinylpyrrolidon und Vinylimidazoliniummethochlorid, Copolymeren aus Methylmethacrylat, tert-Butylaminoethylmethacrylat, 2- Hydroxypropylmethacrylat und Isooctylacrylamid, enthält, **dadurch gekennzeichnet, dass** als desodorierende Komponente ein wässriger oder wässrig-alkoholischer Extrakt von Pflanzenteilen der Birke in einer Menge von wenigstens 0,01 Ges.-% Trockenmasse enthalten ist.

4. Mittel nach Anspruch 3, **dadurch gekennzeichnet, dass** ein Extrakt aus Blättern der Spezies Betula alba (L.) enthalten sind.

5. Verwendung von wäßrigen oder wäßrig-alkoholischen Extrakten von Pflanzenteilen der Birke zur deodorierenden Behandlung der Haut oder der Haare.

## Claims

1. A process for reducing body and environmental odours of the skin or hair by treatment with a care preparation which is not rinsed off with water immediately after use, **characterized in that** the care preparation contains an aqueous or aqueous/alcoholic extract of plant parts of the birch in a quantity of at least 0.01% by weight dry matter.

2. A process as claimed in claim 1, **characterized in that** a care preparation in the form of an aqueous or aqueous/alcoholic spray or an aqueous care foam is used.

3. A preparation for hair care and for reducing body or environmental odours of the hair in the form of an aqueous or aqueous/alcoholic preparation containing a film-forming polymer selected from polyvinyl pyrrolidones and copolymers of vinyl pyrrolidone and vinyl acetate, copolymers of vinyl acetate and crotonic acid, copolymers of methyl vinyl ether and maleic anhydride, quaternized copolymers of vinyl pyrrolidone and dimethylaminoethyl methacrylate, copolymers of vinyl pyrrolidone and vinyl imidazolinium methochloride, copolymers of methyl methacrylate, tert.-butyl aminoethyl methacrylate, 2-hydroxypropyl methacrylate and isooctyl acrylamide, **characterized in that** an aqueous or aqueous/alcoholic extract of plant parts of the birch is present as the deodorizing component in a quantity of at least 0.01% by weight dry matter.

4. A preparation as claimed in claim 3, **characterized in that** an extract of leaves of the species Betula alba (L.) is present.

5. The use of aqueous or aqueous/alcoholic extracts of plant parts of bnthe birch for the deodorizing treatment of the skin or hair.

## Revendications

1. Procédé pour diminuer l'odeur corporelle et de l'environnement sur la peau ou les cheveux par traitement avec un agent de soin qui n'est pas éliminé par rinçage avec de l'eau immédiatement après l'utilisation, **caractérisé en ce que** l'agent de soin contient un extrait aqueux ou aqueux-alcoolique de parties végétales de bouleau en une quantité d'au moins 0,01% en poids de masse sèche.

2. Procédé selon la revendication 1, **caractérisé en ce qu'**on utilise un agent de soin sous forme d'un spray aqueux ou aqueux-alcoolique ou d'une mousse de soin aqueuse.

3. Agent de soin pour cheveux et pour diminuer les odeurs corporelles et de l'environnement sur les cheveux sous forme d'une composition aqueuse ou aqueuse-alcoolique, qui contient un polymère formant un film, choisi parmi les polyvinylpyrrolidones et les copolymères de vinylpyrrolidone et d'acétate de vinyle, les copolymères d'acétate de vinyle et d'acide crotonique, les copolymères de méthylvinyléther et d'anhydride de l'acide maléique, les copolymères quaternisés de vinylpyrrolidone et de méthacrylate de diméthylaminoéthyle, les copolymères de vinylpyrrolidone et de méthochlorure de vinylimidazolinium, les copolymères de méthacrylate de méthyle, de méthacrylate de tert-butylaminoéthyle, de méthacrylate de 2-hydroxypropyle et d'isooctylacrylamide, **caractérisé en ce qu'**il contient comme composant désodorisant un extrait aqueux ou aqueux-alcoolique de parties végétales de bouleau en une quantité d'au moins 0,01% en poids de masse sèche.

4. Agent selon la revendication 3, **caractérisé en ce qu'**il contient un extrait de feuilles de l'espèce Betula alba (L.).

5. Utilisation d'extraits aqueux ou aqueux-alcooliques de parties végétales du bouleau pour le traitement désodorisant de la peau ou des cheveux.
